# FASCICULE DE BREVET EUROPEEN

(11) **EP 0 667 169 B1**
(45) Date de publication et mention de la délivrance du brevet: **07.04.1999**
(21) Numéro de dépôt: 95400042.8
(22) Date de dépôt: 10.01.1995
(51) Int. Cl.: A61M 16/00

(54) **Appareil d'aide à la ventilation d'un patient comportant notamment un mode d'assistance inspiratoire à pression réduite**
Beatmungsgerät mit einem Atemunterstützungsverfahren unter erniedrigtem Druck
Device for respiratory assistance with limited pressure mode of treating support

(30) Priorité: 12.01.1994 FR 9400246
(43) Date de publication de la demande: 16.08.1995
(73) Titulaire: Société d'Applications Industrielles Medicales et Electroniques ( SAIME), 77176 Savigny Le Temple (FR)
(72) Inventeur: Chalvignac, Philippe, F-77123 Noisy sur Ecole (FR)
(74) Mandataire: Kohn, Philippe

(56) Documents cités:
- EP-A- 0 317 417
- EP-A- 0 347 015
- EP-A- 0 425 092
- EP-A- 0 459 647
- EP-A- 0 549 299
- WO-A-89/10768
- FR-A- 2 682 042

## Description

La présente invention concerne un appareil d'assistance à la ventilation d'un patient.

L'invention concerne plus particulièrement un appareil d'aide à la ventilation d'un patient qui respire par des cycles successifs dont chacun comporte une phase inspiratoire et une phase expiratoire, du type comportant notamment un mode d'assistance inspiratoire au cours duquel l'appareil fournit au patient , durant la phase inspiratoire déclenchée par un début d'inspiration du patient, un débit de gaz sous une pression égale à une valeur de consigne et qui comporte:
- une source de gaz sous pression dont un orifice de sortie fournit un débit de gaz sous pression destiné à être transmis aux voies aériennes supérieures du patient;
- un circuit de transmission comportant une conduite principale d'inspiration reliée à l'orifice de sortie de la source de gaz sous pression et à un masque, notamment du type facial, destiné à être porté par le patient, et comportant une conduite secondaire d'expiration reliée au masque et à un orifice d'évacuation ;
- une valve de régulation du débit qui est interposée dans la conduite principale et qui est commandée par un circuit de commande de l'appareil en fonction notamment des valeurs du débit et de la pression du gaz dans la conduite principale d'inspiration mesurées en aval de la valve de régulation, notamment afin de déterminer le début de la phase inspiratoire ; et
- une vanne expiratoire interposée dans la conduite secondaire d'expiration dont l'ouverture est commandée par le circuit de commande de l'appareil, notamment lors de la phase expiratoire de chaque cycle de respiration du patient en mode d'assistance respiratoire.

Un exemple d'un tel type d'appareil est illustré dans les documents WO-A-89.10768 et EP-A-0.425.092

Lorsqu'il est utilisé en mode d'assistance inspiratoire la totalité du débit de gaz sous pression, notamment d'air sous pression, est transmise au masque (porté par le patient) durant chaque phase inspiratoire, la vanne d'expiration étant fermée.

Lors de chaque phase expiratoire, la vanne d'expiration est ouverte et l'orifice d'entrée du masque auquel est reliée la conduite principale est ainsi mis à l'air libre ambiant de manière que le patient puisse expirer librement l'air inspiré lors de la phase inspiratoire précédente.

Lors de cette phase expiratoire, la valve de régulation du débit a pour fonction d'interrompre le débit dans la conduite principale d'inspiration en dérivant par exemple le flux d'air sous pression produit par la source vers l'extérieur de l'appareil.

La valve de régulation du débit doit ainsi permettre d'avoir une pression relative à l'entrée du masque, lors de la phase expiratoire, qui est prédéterminée et qui peut être proche de zéro.

La détection de la phase expiratoire du patient est assurée par le circuit de commande lorsque la pression mesurée dans la conduite principale est supérieure à une valeur de consigne ou lorsque le débit de cette conduite devient inférieur à une valeur de seuil de consigne.

Une conception classique de la valve de régulation décrite dans le document WO-A-89.10768 est telle que la commande de la valve de type "tout ou rien" implique l'utilisation d'un compresseur axial comme source d'air sous pression.

Dans ce document, la variation de pression d'air est ici en effet obtenue en faisant varier la vitesse de rotation du compresseur, ce qui nécessite plusieurs cycles respiratoires pour établir une valeur déterminée de la pression.

De plus, cela entraîne des bruits d'accélération et de décélération du moteur du compresseur qui sont particulièrement nuisibles dans le cas d'un appareil d'assistance respiratoire à domicile.

Dans le document EP-A-0.425.092, le ventilateur décrit et représenté est relié à un masque d'alimentation en air sous pression muni d'ouïes d'aération qui permettent à la fois l'expiration et le passage d'un débit résiduel dans le circuit d'alimentation.

Ces ouïes, munies ou non d'un clapet, doivent être suffisamment grandes pour permettre l'évacuation des gaz expirés même sous une faible pression d'expiration.

Par contre, leur taille est limitée par la nécessité d'avoir un débit de fuite réduit, ce qui entraîne nécessairement que l'expiration ne peut se faire à pression très faible.

L'application du principe décrit dans ce document mis en oeuvre commercialement par la Société RESPIRONICS est connue sous la dénomination commerciale de BIBAP Ventilatory Support System.

Cet appareil ne permet pas d'avoir une pression d'expiration (EPAP) inférieure à 4 mbars, ce qui peut constituer une gêne pour le patient.

Le document EP-A-0.347.015 décrit un appareil respiratoire qui incorpore un dispositif permettant de déterminer un débit de fuite. Ce dispositif est constitué pour l'essentiel par un Venturi et ne comporte aucune pièce mobile qui permette de régler le niveau du débit de fuite. L'adaptation du débit de fuite ne peut être éventuellemnt effectuée qu'au travers de la vanne principale de régulation qui est une vanne à un seul pointeau ne pouvant pas permettre une précision suffisante à très faible débit. De plus, le débit régulé par la vanne pricipale s'ajoute à celui que laissse circuler en permanence le dispositif de débit de fuite, ce qui ne peut qu'augmenter la pression totale au niveau du masque du patient lorsqu'il expire.

L'invention a pour but de proposer un appareil d'aide à la ventilation du type mentionné précédemment qui remédie aux inconvénients qui viennent d'être énoncés.

Dans ce but, l'invention propose un appareil dont la valve de régulation comporte un corps de valve qui délimite une chambre de régulation reliée à un orifice d'alimentation en gaz sous pression, à un orifice de refoulement du gaz sous pression dans la conduite principale d'inspiration et à un orifice de mise à l'air libre commandé par un clapet principal normalement fermé et dont l'ouverture est commandée par un organe de commande des déplacements du clapet pour faire varier la section de passage de l'orifice de mise à l'air libre, caractérisé en ce que la valve comporte un clapet secondaire dont les déplacements sont liés à ceux du clapet principal et qui, en position d'ouverture maximale du clapet principal, obture partiellement l'orifice de refoulement de la valve pour déterminer, lors de chaque phase expiratoire, la valeur d'un débit résiduel dans la conduite principale d'inspiration de gaz sous une pression proche de zéro.

Selon d'autres caractéristiques de l'invention :
- les clapets principal et secondaire sont portés par une tige commune de commande reliée à un électroaimant d'actionnement dont l'alimentation électrique est commandée par le circuit de commande de l'appareil ;
- la valve de régulation comporte une chicane tubulaire axiale qui réduit et décale vers les hautes fréquences le spectre de bruit résultant de l'écoulement des gaz ;
- la chicane tubulaire axiale possède une extrémité sensiblement conique qui, pendant l'expiration, produit un effet venturi permettant de réduire la pression du débit résiduel ;
- la vanne d'expiration est une vanne normalement fermée dont l'ouverture est commandée par le circuit de commande de l'appareil ;
- la vanne d'expiration est une vanne à commande pneumatique alimentée par un distributeur contrôlé par le circuit de commande ;
- le distributeur comporte une entrée branchée à la sortie de la source de pression où règne une haute pression, une sortie reliée à la vanne d'expiration et deux positions, ouverte ou fermée.
- le distributeur possède deux entrées, l'une branchée à la sortie de la source de pression où règne une haute pression, l'autre à l'entrée de la source de pression où règne une dépression, une sortie reliée à la vanne d'expiration, et trois positions, fermée et ouverte sur l'une ou l'autre des deux entrées ;
- la vanne d'expiration reçoit en parallèle au distributeur une commande de pression qui provient de la chambre de sortie de la valve de régulation ;
- la conduite secondaire d'expiration est branchée en dérivation sur la conduite principale d'inspiration en aval des moyens de mesure du débit ;
- la source de gaz sous pression est une turbine à régime de rotation sensiblement constant ;
- la valeur du débit dans la conduite principale est mesurée par un capteur différentiel ;
- la valeur de la pression du débit résiduel est inférieure à 4 mbars et est de préférence égale à 1 mbar.

D'autres caractéristiques et avantages de l'invention apparaîtront à la lecture de la description détaillée qui va suivre pour la compréhension de laquelle on se reportera aux dessins annexés dans lesquels :
- La figure 1 est une vue schématique illustrant la conception d'un appareil conforme aux enseignements de l'invention et qui est illustré en phase inspiratoire ;
- la figure 2 est une vue similaire à celle de la figure 1 sur laquelle l'appareil est illustré en phase expiratoire ;
- la figure 3 est une vue schématique en section d'un exemple de réalisation d'une valve de régulation de débit illustrée dans une position qu'elle occupe en phase inspiratoire ; et
- la figure 4 est une vue similaire à celle de la figure 3 sur laquelle la valve est illustrée dans une position qu'elle occupe en phase expiratoire.
- la figure 5 est une vue schématique illustrant un deuxième mode de réalisation préféré d'un appareil selon l'invention et qui est illustré en phase expiratoire.

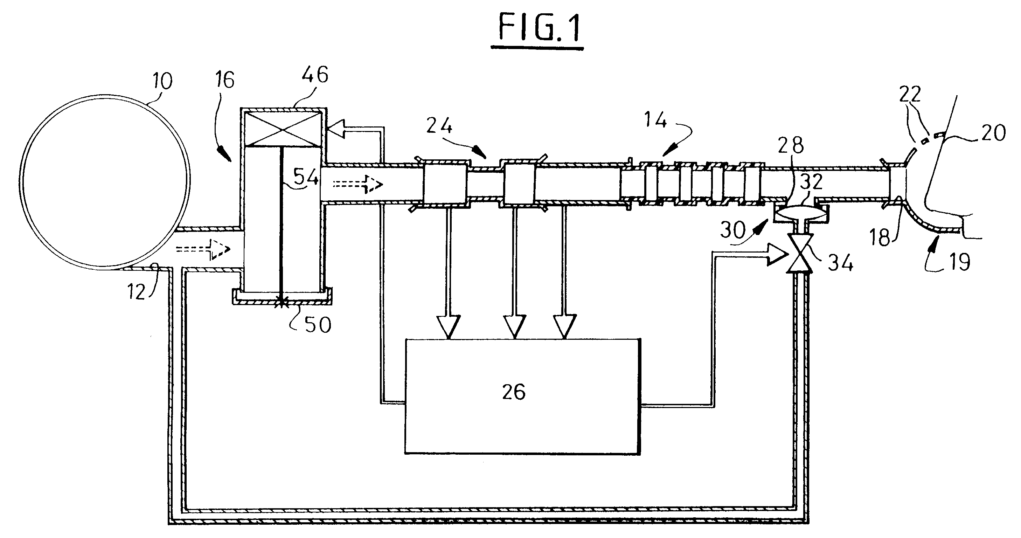

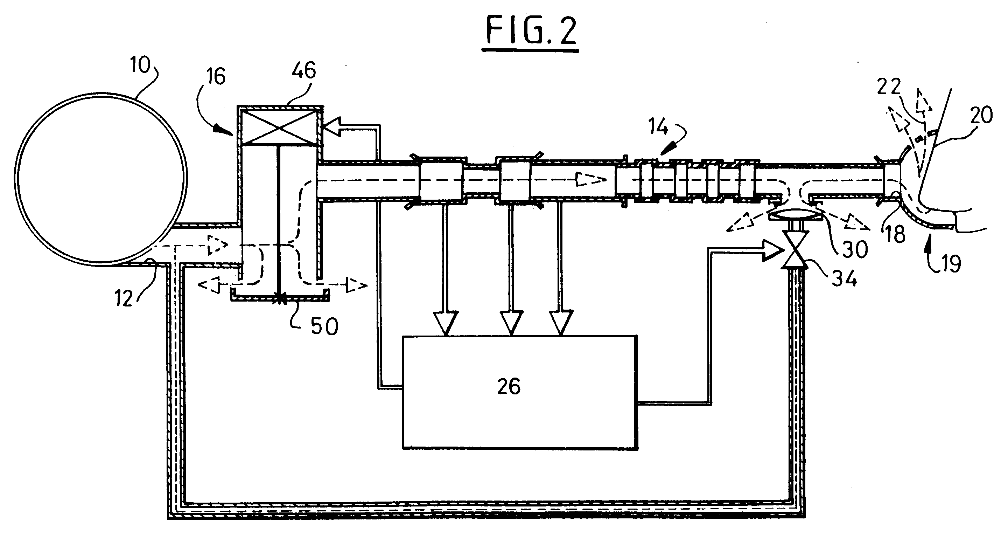

L'appareil illustré sur les figures 1 et 2 comporte un compresseur centrifuge à turbine 10 qui produit un débit d'air sous pression, la turbine 10 étant par exemple à régime de rotation sensiblement constant.

L'orifice de sortie 12 de la turbine 10 est relié à une conduite principale d'inspiration 14 avec interposition d'une valve de régulation de débit 16.

La conduite principale 14 est reliée à l'entrée 18 d'un masque facial 19 porté par un patient 20 et qui comporte une série d'ouïes 22, également appelée ouïes "anti-rebreathing".

Un capteur différentiel 24 de mesure du débit et de la pression dans la conduite d'inspiration 14 est interposé dans cette dernière en aval de la valve de régulation de débit 16. Le capteur 24, d'une conception classique, transmet des signaux représentatifs des valeurs de la pression et du débit mesurées à un circuit électronique de commande 26 de l'appareil.

L'appareil comporte également une conduite principale d'expiration 28 qui est branchée en dérivation sur la conduite principale 14 en aval de la valve 16 et à proximité de l'entrée 18 du masque 19.

Une vanne à commande pneumatique 30 est interposée dans la conduite secondaire d'expiration 28.

Dans l'exemple illustré sur les figures 1 et 2, la vanne 30 est du type à commande pneumatique par l'intermédiaire d'une canalisation d'alimentation en air sous pression 32 qui est reliée en permanence à la turbine 10 et qui gonfle un ballon d'obturation 33.

La vanne 30 est commandée par un distributeur 34 interposé dans la canalisation d'alimentation 32 en amont de la vanne 30 et dont la commande est assurée par le circuit de commande 26.

Le circuit de commande 26 assure également le pilotage de la valve 16 de régulation de débit dont la conception selon l'invention est la suivante.

La valve 16 comporte un corps de valve 36 qui délimite une chambre de régulation 38 dans laquelle débouche un orifice d'alimentation 40 relié à la sortie 12 de la turbine 10.

La chambre 38 dans laquelle est disposée une chicane fixe 42 est reliée à la conduite principale 14 par son orifice de refoulement 44.

La valve 16 comporte un électroaimant 46 dont l'alimentation est commandée par le circuit 26 et qui agit sur un élément de valve mobile 48 monté coulissant dans le corps 36 et qui porte un clapet principal 50 et un clapet secondaire 52 solidaires d'une tige de commande 54 reliée au noyau mobile (non représenté) de l'électroaimant.

Le clapet 50 a pour fonction d'obturer de manière totale ou partielle un orifice 56 d'évacuation formé au fond de la chambre 38 et qui communique.avec l'air libre ambiant.

Le clapet secondaire 52 a pour fonction, en phase expiratoire, de maintenir une section de passage réduite entre la chambre 38 et l'orifice de refoulement 44, de manière à assurer un débit résiduel faible à pression réduite dans la canalisation principale d'inspiration 14.

A cet effet, le clapet secondaire 52 situé en amont de l'orifice 44 maintient un jeu radial "j" avec son siège 60 formé sur la chicane 40, tandis que le clapet principal 50 occupe alors sa position maximale d'ouverture.

La régulation de la pression de sortie au moyen de la valve 16 est réalisée par le circuit de commande 26 qui asservit la valeur de consigne à la fuite résultant de l'ouverture du clapet principal 50. L'accroissement de l'ouverture du clapet principal 50 par rapport à son siège 56 provoque une fuite plus importante et donc une chute de la pression dans la conduite principale 14.

En phase expiratoire et en position fermée du clapet principal 50 de la valve de régulation de débit 16, le débit résiduel fourni par la turbine 10 au patient est de l'ordre d'une dizaine de millilitres par seconde et assure une pression résiduelle proche de zéro, de l'ordre de 1 mbar.

La stabilité du clapet secondaire 52 en phase expiratoire est assurée grâce à la présence des ouïes 20 du masque qui autorisent un débit de fuite du masque sensiblement égal au débit résiduel.

Les ouïes 22 permettent également de supprimer le pic de débit au début de la phase inspiratoire, ce qui permet de réduire le barotraumatisme dans le cas de traitements de patients souffrant de pathologies obstructives des voies aériennes.

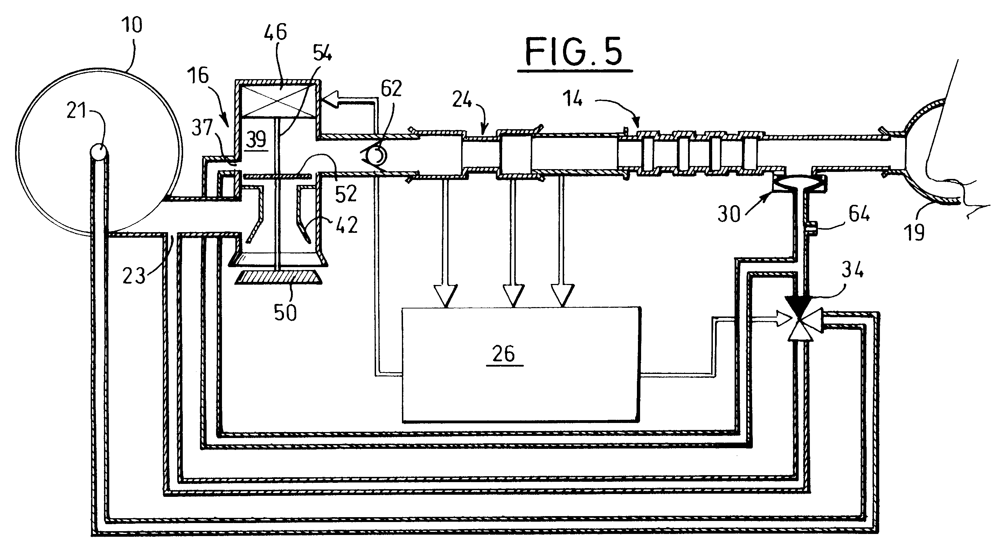

Un deuxième exemple de réalisation de l'invention est présenté sur la figure 5.

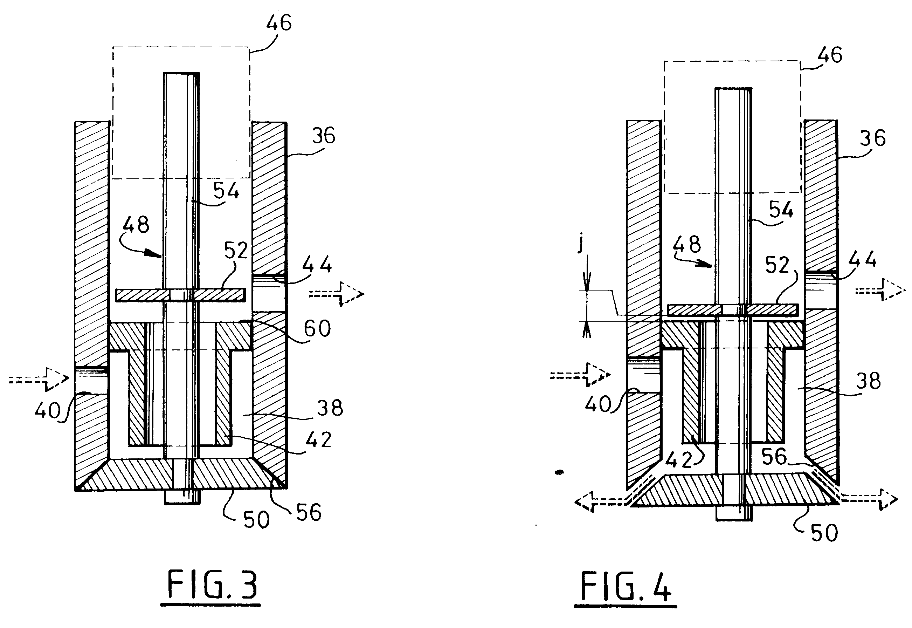

Les éléments représentés sur cette figure, identiques ou similaires à ceux représentés aux figures 1 à 4, sont désignés par les mêmes chiffres de référence.

Dans ce deuxième mode de réalisation, le masque facial 19 par lequel respire le patient est dépourvu d'ouïes d'aération.

Toutefois, diverses dispositions de l'appareil respiratoire permettent de conserver une pression relative proche du zéro lors de la phase expiratoire.

La valve de régulation 16, qui conserve par ailleurs le même fonctionnement que dans le premier exemple de réalisation, possède une chicane tubulaire 42 dont l'extrémité a une forme conique en pavillon de trompette.

La vanne d'expiration à commande pneumatique 30 est reprise du mode de réalisation précédent.

Toutefois, le distributeur 34 est un modèle à double entrée, une entrée étant reliée à la sortie 23 du compresseur 10, l'autre étant reliée à l'entrée 21 d'air du compresseur 10.

Par ailleurs, la vanne d'expiration 30 est reliée de façon permanente à une prise de pression 37 dans la chambre de sortie 39 de la valve de régulation 16.

Enfin, un clapet anti-retour 62 est disposé dans la conduite principale d'inhalation 14, en aval de la valve de régulation 16 et en amont du capteur différentiel 24 de débit de pression.

Pendant l'inspiration, pour maintenir une pression de consigne, l'éléctro-aimant 46 ferme le clapet principal 50, envoyant le débit de gaz dans une chambre de sortie 39 de la valve de régulation 16 et dans la canalisation principale d'inspiration 14.

La prise de pression 37 dans la chambre de sortie 39 de la valve 16 provoque la fermeture de la vanne expiratoire 30.

Toutefois, pour éviter toute fuite indésirable au niveau de cette vanne, le distributeur 34 est commandé par le circuit de commande 26 de manière à fournir à la vanne expiratoire 30 la pression prélevée en sortie 23 de compresseur 10.

La fermeture complète de la vanne 30 est ainsi assurée.

Pendant l'expiration, deux modes de fonctionnement sont notamment envisagés.

Dans un premier mode de fonctionnement, l'électroaimant 46 provoque l'ouverture du clapet principal 50 et la quasi fermeture du clapet secondaire 52 ainsi que cela a été précédemment décrit.

Toutefois, la forme conique de la chicane 42 à pour avantage de réduire encore, par effet venturi, la pression résiduelle dans la chambre de sortie 39.

La prise de pression 37 dans la chambre de sortie 39 de la valve 16 envoie donc une pression proche de zéro à la vanne d'expiration.

De plus, pour assurer l'ouverture complète de la vanne 30, le distributeur 34 est commandé de manière à fournir à la vanne 30 la dépression prélevée en entrée du compresseur 10.

L'ouverture complète de la vanne 30 est ainsi assurée et l'expiration peut se faire à pression d'expiration quasi nulle.

Le clapet anti-retour 62 a notamment pour fonction d'éviter une contamination de la valve de régulation 16 par les gaz expiratoires et d'éviter leur réinhalation.

Dans un deuxième mode de fonctionnement, l'expiration se fait à pression d'expiration non nulle.

Le distributeur 34 est alors fermé et la vanne d'expiration 30 n'est soumise, pour sa commande, qu'à la pression régnant dans la chambre de sortie 39 de la valve de régulation 16, pression qui est régulée par l'ouverture du clapet principal 50 en fonction de la pression mesurée dans la conduite principale d'inspiration 14.

Il est prévu par ailleurs un petit orifice 64 dans le circuit de commande de la vanne 30 qui détermine un débit de fuite dans ce circuit afin d'en assurer la stabilité.

## Revendications

1. Appareil d'aide à la ventilation d'un patient qui respire par des cycles successifs dont chacun comporte une phase inspiratoire et une phase expiratoire, du type comportant notamment un mode d'assistance inspiratoire (AI) au cours duquel l'appareil fournit au patient , durant la phase inspiratoire déclenchée par un début d'inspiration du patient, un débit de gaz sous une pression égale à une valeur de consigne déterminée et qui comporte :
- une source (10) de gaz sous pression dont un orifice de sortie (12) fournit un débit de gaz sous pression destiné à être transmis aux voies aériennes supérieures du patient ;
- un circuit de transmission comportant une conduite principale d'inspiration (14) reliée à l'orifice de sortie (12) de la source de gaz sous pression et à un masque (19), notamment du type facial, destiné à être porté par le patient (20), et comportant une conduite secondaire d'expiration (28) reliée au masque (19) et à un orifice d'évacuation ;
- une valve de régulation du débit (16) qui est interposée dans la conduite principale (14) et qui est commandée par un circuit de commande (26) de l'appareil en fonction notamment des valeurs du débit et de la pression du gaz dans la conduite principale mesurées en aval de la valve de régulation (16), notamment afin de déterminer le début de la phase d'inspiration ;
- une vanne expiratoire (30) interposée dans la conduite secondaire d'expiration (28) dont l'ouverture est commandée par le circuit de commande (26) de l'appareil, notamment lors de la phase expiratoire de chaque cycle de respiration du patient en mode d'assistance respiratoire ; et
ladite valve de régulation (16) comportant un corps de valve (36) qui délimite une chambre de régulation (38) reliée à un orifice (40) d'alimentation en gaz sous pression, à un orifice de refoulement (44,60) du gaz sous pression dans la conduite principale d'inspiration (14) et à un orifice (56) de mise à l'air libre commandé par un clapet principal (50) normalement fermé et dont l'ouverture est commandée par un organe de commande des déplacements du clapet (50) pour faire varier la section de passage de l'orifice de mise à l'air libre (56), caractérisé en ce que ladite valve comporte un clapet secondaire (52) dont les déplacements sont liés à ceux du clapet principal (50) et qui, en position d'ouverture maximale du clapet principal (50), obture partiellement l'orifice de refoulement (44) de la valve (16) pour déterminer, lors de chaque phase expiratoire, la valeur d'un débit résiduel dans la conduite principale d'inspiration (14) de gaz sous une pression proche de zéro.

2. Appareil selon la revendication 1, caractérisé en ce que les clapets principal (50) et secondaire (52) sont portés par une tige commune de commande (54) reliée à un électroaimant (46) d'actionnement dont l'alimentation électrique est commandée par le circuit de commande (26) de l'appareil.

3. Appareil selon la revendication 2, caractérisé en ce que la valve de régulation (16) possède une chicane tubulaire axiale (42) qui réduit et décale vers les hautes fréquences le spectre de bruit résultant de l'écoulement de gaz.

4. Appareil selon la revendication 3, caractérisé en ce que la chicane tubulaire axiale (42) possède une extrémité sensiblement conique qui génère pendant l'expiration un effet venturi permettant de réduire la pression du débit résiduel.

5. Appareil selon l'une quelconque des revendications précédentes, caractérisé en ce que la vanne d'expiration (30) est une vanne normalement fermée dont l'ouverture est commandée par le circuit de commande de l'appareil (26).

6. Appareil selon la revendication 5, caractérisé en ce que la vanne d'expiration (30) est une vanne à commande pneumatique alimentée par un distributeur (34) contrôlé par le circuit de commande (26).

7. Appareil selon la revendication 6, caractérisé en ce que le distributeur possède une entrée branchée à la sortie (23) de la source de pression (10) où règne une haute pression, une sortie reliée à la vanne d'expiration et deux positions, ouverte ou fermée.

8. Appareil selon la revendication 6, caractérisé en ce que le distributeur (34) possède deux entrées, l'une branchée à la sortie (23) de la source de pression (10) où règne une haute pression, l'autre à l'entrée (21) de la source de pression (10) où règne une dépression, une sortie reliée à la vanne d'expiration (30), et trois positions, fermée ou ouverte sur l'une ou l'autre des deux entrées.

9. Appareil selon la revendication 8, caractérisé en ce que la vanne d'expiration (30) reçoit en parallèle au distributeur (34) une commande de pression qui provient de la chambre de sortie (39) de la valve de régulation (16).

10. Appareil selon l'une quelconque des revendications précédentes, caractérisé en ce que la conduite secondaire d'expiration (28) est branchée en dérivation sur la conduite principale d'inspiration (14) en aval des moyens (24) de mesure du débit.

11. Appareil selon l'une quelconque des revendications précédentes, caractérisé en ce que la source de gaz sous pression (10) est une turbine à régime de rotation constant.

12. Appareil selon l'une quelconque des revendications précédentes, caractérisé en ce que la valeur du débit dans la conduite principale est mesurée par un capteur différentiel (24).

13. Appareil selon l'une quelconque des revendications précédentes, caractérisé en ce que la valeur de la pression du débit résiduel est inférieure à 4 mbars et est de préférence sensiblement égale à 1 mbar.

14. Appareil selon l'une quelconque des revendications précédentes, caractérisé en ce qu'il est relié à un masque facial (19) comportant au moins une ouïe (22) de mise en communication de l'intérieur du masque avec l'air ambiant.

## Claims

1. Apparatus for assisting the ventilation of a patient who respires in successive cycles each of which comprises an inhalation phase and an exhalation phase, of the type comprising in particular an inhalation assistance mode (IA) during which the apparatus provides the patient, during the inhalation phase triggered by the start of inhalation by the patient, a flow of gas under a pressure equal to a set value and which comprises:
- a source (10) of gas under pressure of which an outlet orifice (12) provides a flow of gas under pressure intended to be conveyed to the upper airways of the patient;
- a transmission circuit including a main inhalation pipe (14) connected to the outlet orifice (12) of the source of gas under pressure and to a mask (19), in particular of the facial type, designed to be worn by the patient (20), and including a secondary exhalation pipe (28) connected to the mask (19) and to a discharge orifice;
- a flow regulating valve (16) which is interposed in the main pipe (14) and which is controlled by a control circuit (26) of the apparatus as a function in particular of values of the flow rate and of the pressure of the gas in the main pipe measured downstream from the regulating valve (16), so as in particular to determine the start of the inhalation phase;
- an exhalation valve (30) interposed in the secondary exhalation pipe (28), the opening of which is controlled by the control circuit (26) of the apparatus, in particular during the exhalation phase of each respiration cycle of the patient in the assisted respiration mode; and
- the said control valve (16) comprising a valve body (36) which delimits a regulating chamber (38) connected to an orifice (40) supplying a gas under pressure, to an orifice (44, 60) for forcing gas under pressure into the main inhalation pipe (14) and to an orifice (56) for venting to the outside air controlled by a main shut-off valve (50) which is normally closed and the opening of which is controlled by a device controlling movements of the shut-off valve (50) to vary the cross section of the passage of the orifice for venting to the outside air (56), characterized in that the said valve includes a secondary shut-off valve (52) the movements of which are linked to those of the main shut-off valve (50) and which, in the position of maximum opening of the main shut-off valve (50), partially blocks the output orifice (44) of the valve (16) so as to determine, during each exhalation phase, the value of a residual flow in the main inhalation pipe (14) for gas under a pressure close to zero.

2. Apparatus according to claim 1, characterized in that the main shut-off valves (50) and the secondary shut-off valves (52) are carried by common control rod (54) connected to an actuating electromagnet (46), the electrical supply for which is controlled by the control circuit (26) of the apparatus.

3. Apparatus according to claim 2, characterized in that the regulating valve (16) possesses an axial tubular baffle (42) which reduces and shifts the noise spectrum resulting from the outflow of gas towards the high frequencies.

4. Apparatus according to claim 3, characterized in that the axial tubular baffle (42) possesses a substantially conical end which generates, during exhalation, a venturi effect enabling the pressure of the residual flow to be reduced.

5. Apparatus according to any one of the preceding claims, characterized in that the exhalation valve (30) is a normally closed valve, the opening of which is controlled by the control circuit of the apparatus (26).

6. Apparatus according to claim 5, characterized in that the exhalation valve (30) is a pneumatically controlled valve supplied by a distributor (34) operated by the control circuit (26).

7. Apparatus according to claim 6, characterized in that the distributor has an inlet connected to the outlet (23) of the pressure source (10) where the high pressure exists, an outlet connected to the exhalation valve and two positions, open or closed.

8. Apparatus according to claim 6, characterized in that the distributor (34) has two inlets, one connected to the outlet (23) of the pressure source (10) where a high pressure exists, and the other to the inlet (21) of the pressure source (10) where a reduced pressure exists, an outlet connected to the exhalation valve (30), and three positions, closed or open on one or other of the two inlets.

9. Apparatus according to claim 8, characterized in that the exhalation valve (30) receives, at the same time as the distributor (34), a pressure command which comes from the outlet chamber (39) of the regulating valve (16).

10. Apparatus according to any one of the preceding claims, characterized in that the secondary exhalation pipe (28) is connected as a bypass on the main inhalation pipe (14) downstream from the means (24) for measuring the flow rate.

11. Apparatus according to any one of the preceding claims, characterized in that the source of gas under pressure (10) is a turbine with a constant rotational speed.

12. Apparatus according to any one of the preceding claims, characterized in that the value of the flow rate in the main pipe is measured by a differential sensor (24).

13. Apparatus according to any one of the preceding claims, characterized in that the value of the pressure of the residual flow is below 4 mbar and preferably substantially equal to 1 mbar.

14. Apparatus according to any one of the preceding claims, characterized in that it is connected to a face mask (19) having at least one inlet (22) putting the inside of the mask into communication with the ambient air.

## Patentansprüche

1. Gerät zur Unterstützung der Beatmung eines Patienten, der in aufeinanderfolgenden Zyklen atmet, von denen jeder eine Einatmungs- und eine Ausatmungsphase umfasst, von dem Typ, der insbesondere einen Einatmungsunterstützungsmodus (AI) aufweist, in dessen Verlauf das Gerät dem Patienten während der durch einen Beginn der Einatmung des Patienten ausgelösten Einatmungsphase einen Gasfluss unter einem Druck, der gleich einem Solldruck ist, liefert, und welches umfasst:
- eine Quelle (10) für Druckgas, von der eine Ausgangsöffnung (12) einen Druckgasfluss liefert, der vorgesehen ist, um auf die oberen Atmungswege des Patienten übertragen zu werden;
- einen Übertragungskreis, der eine Einatmungs-Hauptleitung (14) umfasst, die mit der Ausgangsöffnung (12) der Druckgasquelle und einer Maske (19), insbesondere einer Gesichtsmaske, verbunden ist, die dazu bestimmt ist, vom Patienten (20) getragen zu werden, und der eine sekundäre Ausatmungsleitung (28) umfasst, die mit der Maske (19) und einer Abführöffnung verbunden ist;
- ein Flussregelventil (16), das in der Hauptleitung (14) zwischengeschaltet ist und durch eine Steuerschaltung (26) des Geräts gesteuert ist, insbesondere in Abhängigkeit von den Werten des Flusses und des Drucks des Gases in der Hauptleitung, gemessen stromabwärts vom Regelventil (16), insbesondere um den Beginn der Einatmungsphase zu bestimmen;
- ein Ausatmungsventil, (30), das in der sekundären Ausatmungsleitung (28) zwischengeschaltet ist und dessen Öffnen durch die Steuerschaltung (26) des Geräts gesteuert ist, insbesondere in der Ausatmungsphase jedes Atmungszyklus des Patienten im Atmungsunterstützungsmodus;
wobei das Regelventil (16) einen Ventilkörper (36) umfasst, der eine Regelkammer (38) begrenzt, die verbunden ist mit einer Druckgasversorgungsöffnung (40), einer Verdrängungsöffnung (44, 60) des Druckgases in die Einatmungs-Hauptleitung (14) und einer Öffnung (56) zur Verbindung mit der freien Luft, die durch eine normalerweise geschlossene Hauptklappe (50) gesteuert ist, deren Öffnen durch ein Organ zum Steuern der Bewegungen der Klappe (50) gesteuert ist, um den Durchgangsquerschnitt der Öffnung (56) zur Verbindung mit der freien Luft zu variieren,
**dadurch gekennzeichnet**, dass das Ventil eine sekundäre Klappe (52) aufweist, deren Bewegungen mit denen der Hauptklappe (50) verknüpft sind, und das in Position maximaler Öffnung der Hauptklappe (50) die Verdrängungsöffnung (44) des Ventils (16) teilweise verschließt, um in jeder Ausatmungsphase den Wert einen Restflusses von Druckgas in der Einatmungs-Hauptleitung (14) von nahe Null festzulegen.

2. Gerät nach Anspruch 1, dadurch gekennzeichnet, dass die Hauptklappe (50) und die sekundäre Klappe (52) von einer gemeinsamen Steuerstange (54) getragen sind, die mit einem Betätigungs-Elektromagneten (46) verbunden ist, dessen Stromversorgung durch die Steuerschaltung (26) des Geräts gesteuert ist.

3. Gerät nach Anspruch 2, dadurch gekennzeichnet, dass das Regelventil (16) einen rohrförmigen axialen Krümmer (42) aufweist, der das Geräuschspektrum, das aus der Gasströmung resultiert, verringert und zu hohen Frequenzen verschiebt.

4. Gerät nach Anspruch 3, dadurch gekennzeichnet, dass der rohrförmige axiale Krümmer (452) ein im wesentlichen konisches Ende besitzt, das während der Ausatmung einen Düseneffekt hervorruft, der es erlaubt, den Druck des Restflusses zu verringern.

5. Gerät nach einem beliebigen der vorhergehenden Ansprüche, dadurch gekennzeichnet, dass das Ausatmungsventil (30) ein normalerweise geschlossenes Ventil ist, dessen Öffnen durch die Steuerschaltung (26) des Geräts gesteuert ist.

6. Gerät nach Anspruch 5, dadurch gekennzeichnet, dass das Ausatmungsventil (30) ein Ventil mit pneumatischer Steuerung ist, das von einem durch die Steuerschaltung (26) gesteuerten Verteiler (34) versorgt wird.

7. Gerät nach Anspruch 6, dadurch gekennzeichnet, dass der Verteiler einen an den Ausgang (23) der Druckquelle (10), wo ein hoher Druck herrscht, angeschlossenen EIngang, einen an das Ausatmungsventil angeschlossenen Ausgang und zwei Positionen, offen oder geschlossen, hat.

8. Gerät nach Anspruch 6, dadurch gekennzeichnet, dass der Verteiler (34) zwei Eingänge, von denen einer an den Ausgang (23) der Druckquelle (10), wo ein hoher Druck herrscht, angeschlossen ist, und der andere an den Eingang (21) der Druckquelle, wo ein Unterdruck herrscht, angeschlossen ist, einen an das Ausatmungsventil (30) angeschlossenen Ausgang und drei Positionen, geschlossen oder an einem der zwei Eingänge offen, hat.

9. Gerät nach Anspruch 8, dadurch gekennzeichnet, dass das Ausatmungsventil (30) parallel zum Verteiler (34) eine Drucksteuerung empfängt, die von der Ausgangskammer (39) des Regelventils (16) kommt.

10. Gerät nach einem beliebigen der vorhergehenden Ansprüche, dadurch gekennzeichnet, dass die sekundäre Ausatmungsleitung (28) als Abzweig an die Einatmungs-Hauptleitung stromabwärts von den Mitteln zu Messen des Flusses (24) angeschlossen ist.

11. Gerät nach einem beliebigen der vorhergehenden Ansprüche, dadurch gekennzeichnet, dass die Druckgasquelle (10) eine Turbine in Konstantdrehzahl-Betrieb ist.

12. Gerät nach einem beliebigen der vorhergehenden Ansprüche, dadurch gekennzeichnet, dass der Wert des Flusses in der Hauptleitung durch einen differentiellen Aufnehmer (24) gemessen wird.

13. Gerät nach einem beliebigen der vorhergehenden Ansprüche, dadurch gekennzeichnet, dass der Wert des Drucks des Restflusses kleiner als 4 mbar und vorzugsweise im wesentlichen gleich 1 mbar ist.

14. Gerät nach einem beliebigen der vorhergehenden Ansprüche, dadurch gekennzeichnet, dass es an eine Gesichtsmaske (19) angeschlossen ist, die wenigstens eine Öffnung zum In-Verbindung-Setzen mit der Umgebungsluft aufweist.
